# EUROPEAN PATENT SPECIFICATION

(11) **EP 2 658 946 B1**
(45) Date of publication and mention of the grant of the patent: **07.10.2015**
(21) Application number: 11852890.0
(22) Date of filing: 09.12.2011
(51) Int. Cl.: C09K 8/584, C07C 309/20, E21B 43/16, C09K 8/58

(54) **METHOD AND COMPOSITION FOR ENHANCED HYDROCARBONS RECOVERY FROM A FORMATION CONTAINING A CRUDE OIL**
VERFAHREN UND ZUSAMMENSETZUNG FÜR VERSTÄRKTE KOHLENWASSERSTOFFGEWINNUNG AUS EINER ROHÖLHALTIGEN FORMATION
PROCÉDÉ ET COMPOSITION POUR UNE MEILLEURE EXTRACTION DES HYDROCARBURES DANS UNE FORMATION CONTENANT DU PÉTROLE BRUT

(30) Priority: 29.12.2010 US 201061427923 P
(43) Date of publication of application: 06.11.2013
(73) Proprietor: Shell Oil Company, Houston, TX 77252-2463 (US); Shell Internationale Research Maatschappij B.V., 2596 HR The Hague (NL); William Marsh Rice University, Houston, TX 77005 (US)
(72) Inventor: BARNES, Julian Richard, NL-1031 HW Amsterdam (NL); ELLISON, Robert Hardy, Katy, TX 77494 (US); PUERTO, Maura, Houston, TX 77077 (US); RANEY, Kirk Herbert, Houston, TX 77094 (US); SMIT,Johan Paul, NL-1031 HW Amsterdam (NL)
(74) Representative: Matthezing, Robert Maarten
(86) International application number: PCT/US2011/064088
(87) International publication number: WO 2012/091881

(56) References cited:
- GB-A- 1 389 312
- GB-A- 2 111 559
- JP-A- S5 984 971
- US-A- 3 755 203
- US-A- 3 852 221
- US-A- 4 576 232
- US-A- 4 813 483
- US-A- 4 852 653
- US-A- 4 852 653
- US-A- 5 110 487
- US-A- 6 165 958
- US-A1- 2009 163 755
- US-B1- 6 566 319

## Description

### Field of the Invention

The present invention generally relates to methods for recovery of hydrocarbons from hydrocarbon-bearing formations. More particularly, embodiments described herein relate to methods of enhanced hydrocarbon recovery and to compositions useful therein.

### Background of the Invention

Hydrocarbons may be recovered from hydrocarbon-bearing formations by penetrating the formation with one or more wells. Hydrocarbons may flow to the surface through the wells. Conditions (e.g., permeability, hydrocarbon concentration, porosity, temperature, pressure, amongst others) of the hydrocarbon containing formation may affect the economic viability of hydrocarbon production from the hydrocarbon containing formation. A hydrocarbon-bearing formation may have natural energy (e.g., gas, water) to aid in mobilizing hydrocarbons to the surface of the hydrocarbon containing formation. Natural energy may be in the form of water. Water may exert pressure to mobilize hydrocarbons to one or more production wells. Gas may be present in the hydrocarbon-bearing formation (reservoir) at sufficient pressures to mobilize hydrocarbons to one or more production wells. The natural energy source may become depleted over time. Supplemental recovery processes may be used to continue recovery of hydrocarbons from the hydrocarbon containing formation. Examples of supplemental processes include waterflooding, polymer flooding, alkali flooding, thermal processes, solution flooding or combinations thereof.

In chemical enhanced oil recovery (EOR) the mobilization of residual oil saturation is achieved through surfactants which generate a sufficiently (ultra) low crude oil / water interfacial tension (IFT) to give a capillary number large enough to overcome capillary forces and allow the oil to flow (I. Chatzis and N. R. Morrows, "Correlation of capillary number relationship for sandstone" SPE Journal, Vol 29, pp 555-562, 1989). However, reservoirs have different characteristics (crude oil type and composition, temperature and the water composition - salinity, hardness) and it is desirable that the structures of added surfactant(s) be matched to these conditions to achieve a low IFT. In addition, a promising surfactant must fulfill other important criteria including low rock retention, compatibility with polymer, thermal and hydrolytic stability and acceptable cost.

Compositions and methods for enhanced hydrocarbons recovery utilizing ;an alpha olefin sulfate-containing surfactant component are known. U.S. Patents 4,488,976 and 4,537,253 describe enhanced oil or recovery compositions containing such a component. Compositions and methods for enhanced hydrocarbons recovery utilizing internal olefin sulfonates are also known. Such a surfactant composition is described in U.S. Patent 4,597,879. The compositions described in the foregoing patents have the disadvantages that brine solubility and divalent ion tolerance are insufficient at certain reservoir conditions.

U.S. Patent 4,979,564 describes the use of internal olefin sulfonates in a method for enhanced oil recovery using low tension viscous water flood. An example of a commercially available material described as being useful was ENORDET IOS 1720, a product of Shell Oil Company identified as a sulfonated C₁₇₋₂₀ internal olefin sodium salt. This material has a low degree of branching. U.S. Patent 5,068,043 describes a petroleum acid soap-containing surfactant system for waterflooding wherein a cosurfactant comprising a C₁₇₋₂₀ or a C₂₀₋₂₄ internal olefin sulfonate was used.

### Summary of the Invention

The invention provides a method of treating a formation containing crude oil, comprising: (a) providing a hydrocarbon recovery composition to at least a portion of the crude oil containing formation, wherein the composition comprises at least 10 wt% of vinylidene olefin sulfonate, and wherein the hydrocarbon recovery composition is provided to the crude oil containing formation by first admixing it with water and/or brine from the formation from which crude oil is to be extracted to form an injectable fluid, wherein the vinylidene olefin sulfonate comprises from 0.05 to 1.0 wt%, preferably from 0.1 to 0.8 wt% of the injectable fluid, and then injecting the injectable fluid into the formation; and (b) allowing the composition to interact with hydrocarbons in the crude oil containing formation.

### Brief Description of the Drawings

FIG. 1 depicts an embodiment of treating a hydrocarbon containing formation.
FIG. 2 depicts an embodiment of treating a hydrocarbon containing formation.

### Detailed Description of Embodiments

Hydrocarbons may be produced from hydrocarbon formations through wells penetrating a hydrocarbon containing formation. "Hydrocarbons" are generally defined as molecules formed primarily of carbon and hydrogen atoms such as oil and natural gas. Hydrocarbons may also include other elements, such as, but not limited to, halogens, metallic elements, nitrogen, oxygen and/or sulfur. Hydrocarbons derived from a hydrocarbon formation may include, but are not limited to, kerogen, bitumen, pyrobitumen, asphaltenes, resins, saturates, naphthenic acids, oils or combinations thereof. Hydrocarbons may be located within or adjacent to mineral matrices within the earth. Matrices may include, but are not limited to, sedimentary rock, sands, silicilytes, carbonates, diatomites and other porous media.

A "formation" includes one or more hydrocarbon containing layers, one or more non-hydrocarbon layers, an overburden and/or an underburden. An "overburden" and/or an "underburden" includes one or more different types of impermeable materials. For example, overburden/underburden may include rock, shale, mudstone, or wet/tight carbonate (i.e., an impermeable carbonate without hydrocarbons). For example, an underburden may contain shale or mudstone. In some cases, the overburden/underburden may be somewhat permeable. For example, an underburden may be composed of a permeable mineral such as sandstone or limestone. In some embodiments, at least a portion of a hydrocarbon containing formation may exist at less than or more than 1000 feet (304.8 m) below the earth's surface.

Properties of a hydrocarbon containing formation may affect how hydrocarbons flow through an underburden/overburden to one or more production wells. Properties include, but are not limited to, mineralogy, porosity, permeability, pore size distribution, surface area, salinity or temperature of formation. Overburden/underburden properties in combination with hydrocarbon properties, such as, capillary pressure (static) characteristics and relative permeability (flow) characteristics may affect mobilization of hydrocarbons through the hydrocarbon containing formation.

Permeability of a hydrocarbon containing formation may vary depending on the formation composition. A relatively permeable formation may include heavy hydrocarbons entrained in, for example, sand or carbonate. "Relatively permeable," as used herein, refers to formations or portions thereof, that have an average permeability of 10 millidarcy (9.9 x 10⁻³ square micrometers) or more. "Relatively low permeability" as used herein, refers to formations or portions thereof that have an average permeability of less than about 10 millidarcy (9.9 x 10⁻³ square micrometers). One darcy is equal to about 0.99 square micrometers. An impermeable portion of a formation generally has a permeability of less than about 0.1 millidarcy (0.099 x 10⁻³ square micrometers). In some cases, a portion or all of a hydrocarbon portion of a relatively permeable formation may include predominantly heavy hydrocarbons and/or tar with no supporting mineral grain framework and only floating (or no) mineral matter (e.g., asphalt lakes).

Fluids (e.g., gas, water, hydrocarbons or combinations thereof) of different densities may exist in a hydrocarbon containing formation. A mixture of fluids in the hydrocarbon containing formation may form layers between an underburden and an overburden according to fluid density. Gas may form a top layer, hydrocarbons may form a middle layer and water may form a bottom layer in the hydrocarbon containing formation. The fluids may be present in the hydrocarbon containing formation in various amounts. Interactions between the fluids in the formation may create interfaces or boundaries between the fluids. Interfaces or boundaries between the fluids and the formation may be created through interactions between the fluids and the formation. Typically, gases do not form boundaries with other fluids in a hydrocarbon containing formation. In an embodiment, a first boundary may form between a water layer and underburden. A second boundary may form between a water layer and a hydrocarbon layer. A third boundary may form between hydrocarbons of different densities in a hydrocarbon containing formation. Multiple fluids with multiple boundaries may be present in a hydrocarbon containing formation, in some embodiments. It should be understood that many combinations of boundaries between fluids and between fluids and the overburden/underburden may be present in a hydrocarbon containing formation.

Production of fluids may perturb the interaction between fluids and between fluids and the overburden/underburden. As fluids are removed from the hydrocarbon containing formation, the different fluid layers may mix and form mixed fluid layers. The mixed fluids may have different interactions at the fluid boundaries. Depending on the interactions at the boundaries of the mixed fluids, production of hydrocarbons may become difficult. Quantification of the interactions (e.g., energy level) at the interface of the fluids and/or fluids and overburden/underburden may be useful to predict mobilization of hydrocarbons through the hydrocarbon containing formation.

Quantification of energy required for interactions (e.g., mixing) between fluids within a formation at an interface may be difficult to measure. Quantification of energy levels at an interface between fluids may be determined by generally known techniques (e.g., spinning drop tensionmeter, Langmuir trough). Interaction energy requirements at an interface may be referred to as interfacial tension. "Interfacial tension" as used herein, refers to a surface free energy that exists between two or more fluids that exhibit a boundary. A high interfacial tension value (e.g., greater than about 10 dynes/cm (10 x 10⁻⁵ N/cm)) may indicate the inability of one fluid to mix with a second fluid to form a fluid emulsion. As used herein, an "emulsion" refers to a dispersion of one immiscible fluid into a second fluid by addition of a composition that reduces the interfacial tension between the fluids to achieve stability. The inability of the fluids to mix may be due to high surface interaction energy between the two fluids. Low interfacial tension values (e.g., less than about 1 dyne/cm (1 x 10⁻⁵ N/cm)) may indicate less surface interaction between the two immiscible fluids. Less surface interaction energy between two immiscible fluids may result in the mixing of the two fluids to form an emulsion. Fluids with low interfacial tension values may be mobilized to a well bore due to reduced capillary forces and subsequently produced from a hydrocarbon containing formation.

Fluids in a hydrocarbon containing formation may wet (e.g., adhere to an overburden/underburden or spread onto an overburden/underburden in a hydrocarbon containing formation). As used herein, "wettability" refers to the preference of a fluid to spread on or adhere to a solid surface in a formation in the presence of other fluids. In an embodiment, hydrocarbons may adhere to sandstone in the presence of gas or water. An overburden/underburden that is substantially coated by hydrocarbons may be referred to as "oil wet." An overburden/underburden may be oil wet due to the presence of polar and/or or surface-active components (e.g., asphaltenes) in the hydrocarbon containing formation. Formation composition (e.g., silica, carbonate or clay) may determine the amount of adsorption of hydrocarbons on the surface of an overburden/underburden. In some embodiments, a porous and/or permeable formation may allow hydrocarbons to more easily wet the overburden/underburden. A substantially oil wet overburden/underburden may inhibit hydrocarbon production from the hydrocarbon containing formation. In certain embodiments, an oil wet portion of a hydrocarbon containing formation may be located at less than or more than 1000 feet (304.8 m) below the earth's surface.

A hydrocarbon formation may include water. Water may interact with the surface of the underburden. As used herein, "water wet" refers to the formation of a coat of water on the surface of the overburden/underburden. A water wet overburden/underburden may enhance hydrocarbon production from the formation by preventing hydrocarbons from wetting the overburden/underburden. In certain embodiments, a water wet portion of a hydrocarbon containing formation may include minor amounts of polar and/or surface-active components.

Water in a hydrocarbon containing formation may contain minerals (e.g., minerals containing barium, calcium, or magnesium) and mineral salts (e.g., sodium chloride, potassium chloride, magnesium chloride). Water salinity, pH and/or water hardness of water in a formation may affect recovery of hydrocarbons in a hydrocarbon containing formation. As used herein "salinity" refers to an amount of dissolved solids in water. "Water hardness," as used herein, refers to a concentration of divalent ions (e.g., calcium, magnesium) in the water. Water salinity and hardness may be determined by generally known methods (e.g., conductivity, titration). As water salinity increases in a hydrocarbon containing formation, interfacial tensions between hydrocarbons and water may be increased and the fluids may become more difficult to produce.

A hydrocarbon containing formation may be selected for treatment based on factors such as, but not limited to, thickness of hydrocarbon containing layers within the formation, assessed liquid production content, location of the formation, salinity content of the formation, temperature of the formation, and depth of hydrocarbon containing layers. Initially, natural formation pressure and temperature may be sufficient to cause hydrocarbons to flow into well bores and out to the surface. Temperatures in a hydrocarbon containing formation may range from about 0°C to about 300°C though a typical maximum reservoir temperature for crude oil enhanced oil recovery is about 150°C. The composition of the present invention is particularly advantageous when used at high temperature because the vinylidene olefin sulfonate is stable at such temperatures. As hydrocarbons are produced from a hydrocarbon containing formation, pressures and/or temperatures within the formation may decline. Various forms of artificial lift (e.g., pumps, gas injection) and/or heating may be employed to continue to produce hydrocarbons from the hydrocarbon containing formation. Production of desired hydrocarbons from the hydrocarbon containing formation may become uneconomical as hydrocarbons are depleted from the formation.

Mobilization of residual hydrocarbons retained in a hydrocarbon containing formation may be difficult due to viscosity of the hydrocarbons and capillary effects of fluids in pores of the hydrocarbon containing formation. As used herein "capillary forces" refers to attractive forces between fluids and at least a portion of the hydrocarbon containing formation. In an embodiment, capillary forces may be overcome by increasing the pressures within a hydrocarbon containing formation. In other embodiments, capillary forces may be overcome by reducing the interfacial tension between fluids in a hydrocarbon containing formation. The ability to reduce the capillary forces in a hydrocarbon containing formation may depend on a number of factors, including, but not limited to, the temperature of the hydrocarbon containing formation, the salinity of water in the hydrocarbon containing formation, and the composition of the hydrocarbons in the hydrocarbon containing formation.

As production rates decrease, additional methods may be employed to make a hydrocarbon containing formation more economically viable. Methods may include adding sources of water (e.g., brine, steam), gases, polymers, monomers or any combinations thereof to the hydrocarbon formation to increase mobilization of hydrocarbons.

In an embodiment, a hydrocarbon containing formation may be treated with a flood of water. A waterflood may include injecting water into a portion of a hydrocarbon containing formation through injections wells. Flooding of at least a portion of the formation may water wet a portion of the hydrocarbon containing formation. The water wet portion of the hydrocarbon containing formation may be pressurized by known methods and a water/hydrocarbon mixture may be collected using one or more production wells. The water layer, however, may not mix with the hydrocarbon layer efficiently. Poor mixing efficiency may be due to a high interfacial tension between the water and hydrocarbons.

Production from a hydrocarbon containing formation is enhanced by treating the hydrocarbon containing formation with a polymer and/or monomer that may mobilize hydrocarbons to one or more production wells. The polymer and/or monomer may reduce the mobility of the water phase in pores of the hydrocarbon containing formation. The reduction of water mobility may allow the hydrocarbons to be more easily mobilized through the hydrocarbon containing formation. Polymers include, but are not limited to, polyacrylamides, partially hydrolyzed polyacrylamide, polyacrylates, ethylenic copolymers, biopolymers, carboxymethylcellulose, polyvinyl alcohol, polystyrene sulfonates, polyvinylpyrrolidone, AMPS (2-acrylamide-2-methyl propane sulfonate) or combinations thereof. Examples of ethylenic copolymers include copolymers of acrylic acid and acrylamide, acrylic acid and lauryl acrylate, lauryl acrylate and acrylamide. Examples of biopolymers include xanthan gum and guar gum. In some embodiments, polymers may be cross linked in situ in a hydrocarbon containing formation. In other embodiments, polymers may be generated in situ in a hydrocarbon containing formation. Polymers and polymer preparations for use in oil recovery are described in U.S. Patent No. 6,427,268 to Zhang et al., entitled "Method For Making Hydrophobically Associative Polymers, Methods of Use and Compositions;" U.S. Patent No. 6,439,308 to Wang, entitled "Foam Drive Method;" U.S. Patent No. 5,654,261 to Smith, entitled, "Permeability Modifying Composition For Use In Oil Recovery;" U.S. Patent No. 5,284,206 to Surles et al., entitled "Formation Treating;" U.S. Patent 5,199,490 to Surles et al., entitled "Formation Treating" and U.S. Patent No. 5,103,909 to Morgenthaler et al., entitled "Profile Control In Enhanced Oil Recovery."

### The Hydrocarbon Recovery Composition

In an embodiment, a hydrocarbon recovery composition may be provided to the hydrocarbon containing formation. In this invention the composition comprises a particular vinylidene olefin sulfonate or blend of vinylidene olefin sulfonates. Vinylidene olefin sulfonates are chemically suitable for EOR.

As discussed above in detail, this invention is particularly useful in hydrocarbon containing formations which contain crude oil. The hydrocarbon recovery composition of this invention is designed to produce the best vinylidene olefin sulfonate recovery composition for these crude oils containing formations and for the brine found in these formations. The preferred composition comprises a C16, C20 or C24 vinylidene olefin sulfonate.

A vinylidene olefin is an olefin of the general structure of a 2-alkyl-1-alkene. In an embodiment, the hydrocarbon recovery composition may comprise from about 1 to about 75 wt% of the vinylidene olefin sulfonate or blend containing it, preferably from about 10 to about 40 wt% and more preferably from about 20 to about 30 wt%. In an embodiment, a hydrocarbon containing composition may be produced from a hydrocarbon containing formation. The hydrocarbon containing composition may include any combination of hydrocarbons, the vinylidene olefin sulfonate described above, a solubilizing agent, methane, water, asphaltenes, carbon monoxide and ammonia.

The remainder of the composition may include, but is not limited to, water, low molecular weight alcohols, organic solvents, alkyl sulfonates, aryl sulfonates, brine or combinations thereof. Low molecular weight alcohols include, but are not limited to, methanol, ethanol, propanol, isopropyl alcohol, *tert*-butyl alcohol, *sec*-butyl alcohol, butyl alcohol, *tert*-amyl alcohol or combinations thereof. Organic solvents include, but are not limited to, methyl ethyl ketone, acetone, lower alkyl cellosolves, lower alkyl carbitols or combinations thereof.

### Manufacture of the Hydrocarbon Recovery Composition

The vinylidene olefins that are used to make the vinylidene olefin sulfonates of the present invention may be made by dimerization of alpha olefins. Alpha olefins are defined as an olefin whose double bond is located at a terminal carbon atom. The alpha olefins may include any alpha olefin with from 4 to 18 carbon atoms. The alpha olefins preferably comprise alpha olefins with from 6 to 16 carbon atoms. More preferred alpha olefins have from 6 to 12 carbon atoms.

The dimerization may be carried out with a single alpha olefin or a blend of alpha olefins. When a single alpha olefin is used, it is preferably a C6, C8, C10 or C12 alpha olefin. When a blend of alpha olefins is used, any combination of alpha olefins may be used.

Physical properties of the final product are typically impacted by the starting materials selected, so the use of some alpha olefins will result in more preferred final products. Some examples of possible blends of alpha olefins are C4 with C8; C4 with C10; C4 with C12; C4 with C14; C4 with C16; C6 with C8; C6 with C10; C6 with C12; C6 with C14; C6 with C18; C8 with C10; C8 with C12; C10 with C12; and C12 with C14. Further it is possible to envision a blend of more than two alpha olefins that could be used to produce suitable products.

The process will be described below in respect to using a single alpha olefin, C8, but this process applies equally to the other single alpha olefins and the blends of alpha olefins described above.

The first step of the process is to dimerize 1-octene to produce 2-hexyl-1-decene. The 2-hexyl-1-decene is a vinylidene olefin that may also be referred to as 7-methylene pentadecane. There are a number of processes for carrying out this dimerization; for example, the processes described in US 4,658,078; US 4,973,788; and US 7,129,197. Dimerization using a metallocene catalyst results in a single vinylidene compound being formed. The product may be distilled, if desired, to remove unreacted monomer and any trimer or higher oligomers that may have formed or the product may be directly used in the next step.

A process which can be used to make vinylidene olefin sulfonates for use in the present invention comprises reacting in a film reactor a vinylidene olefin as described above with a sulfonating agent in a mole ratio of sulfonating agent to vinylidene olefin of 1:1 to 1.25:1 while cooling the reactor with a cooling means having a temperature not exceeding 35 °C, directly neutralizing the obtained reaction product of the sulfonating step and, without extracting the unreacted vinylidene olefin, hydrolyzing the neutralized reaction product.

In the preparation of the sulfonates derived from vinylidene olefins, the vinylidene olefins are reacted with a sulfonating agent, which may be sulfur trioxide, sulfuric acid, or oleum, with the formation of beta-sultone. The film reactor is preferably a falling film reactor.

The reaction products are neutralized and hydrolyzed. When alpha- or internal-olefins are sulfonated,the beta-sultones are converted upon aging into gamma-sultones which then subsequently are converted into delta-sultones. After neutralization and hydrolysis, a mixture of alkene sulfonates and hydroxyalkane sulfonates are obtained. In contrast, the beta-sultones obtained after sulfonating vinylidene olefins do not isomerize to gamma and delta forms but are converted directly to alkene sulfonates upon neutralization. No hydroxyalkane sulfonates are formed. Thus, vinylidene olefin sulfonates contain much fewer isomers than corresponding alpha and internal olefin sulfonates of the same average carbon number. This high purity would have the beneficial property of reducing or eliminating undesired chromatographic separation of surfactant components in the oil reservoir. The vinylidene olefin sulfonate may also give a lower interfacial tension (IFT) and better EOR performance than a corresponding internal olefin sulfonate, when the molecule is matched to a particular reservoir condition.

The cooling means, which is preferably water, has a temperature not exceeding 35 °C, especially a temperature in the range of from 0 to 25 °C. Depending upon the circumstances, lower temperatures may be used as well.

The reaction mixture is then fed to a neutralization hydrolysis unit. The neutralization/hydrolysis is carried out with a water soluble base, such as sodium hydroxide or sodium carbonate. The corresponding bases derived from potassium or ammonium are also suitable. The neutralization of the reaction product from the falling film reactor is generally carried out with excessive base, calculated on the acid component. Generally, neutralization is carried out at a temperature in the range of from 0 to 80 °C. Hydrolysis may be carried out at a temperature in the range of from 100 to 250 °C, preferably 130 to 200 °C. The hydrolysis time generally may be from 5 minutes to 4 hours. Alkaline hydrolysis may be carried out with hydroxides, carbonates, bicarbonates of (earth) alkali metals, and amine compounds.

This process may be carried out batchwise, semi-continuously, or continuously. The reaction is generally performed in a falling film reactor which is cooled by flowing a cooling means at the outside walls of the reactor. At the inner walls of the reactor, the vinylidene olefin flows in a downward direction. Sulfur trioxide is diluted with a stream of nitrogen, air, or any other inert gas into the reactor. The concentration of sulfur trioxide generally is between 2 and 5 percent by volume based on the volume of the carrier gas. In the preparation of vinylidene olefin sulfonates derived from the vinylidene olefins of the present invention, it is required that in the neutralization hydrolysis step very intimate mixing of the reactor product and the aqueous base is achieved. This can be done, for example, by efficient stirring or the addition of a polar cosolvent (such as a lower alcohol) or by the addition of a phase transfer agent.

### Injection of the Hydrocarbon Recovery Composition

The hydrocarbon recovery composition may interact with hydrocarbons in at least a portion of the hydrocarbon containing formation. Interaction with the hydrocarbons may reduce an interfacial tension of the hydrocarbons with one or more fluids in the hydrocarbon containing formation. In other embodiments, a hydrocarbon recovery composition may reduce the interfacial tension between the hydrocarbons and an overburden/underburden of a hydrocarbon containing formation. Reduction of the interfacial tension may allow at least a portion of the hydrocarbons to mobilize through the hydrocarbon containing formation.

The ability of a hydrocarbon recovery composition to reduce the interfacial tension of a mixture of hydrocarbons and fluids may be evaluated using known techniques. In an embodiment, an interfacial tension value for a mixture of hydrocarbons and water may be determined using a spinning drop tensionmeter.

Due to the well-established relationship between micro-emulsion phase behavior and IFT, it is common in the industry to screen surfactants and their formulations for low IFT behavior through laboratory-based oil / water phase behavior tests as described in D.B Levitt et al, "Identification and Evaluation of High Performance EOR Surfactants", SPE 100089. In micro-emulsion phase tests the optimal salinity is the point where equal amounts of oil and water are solubilised in the middle phase microemulsion, known as Winsor type III. The oil solubilisation parameter is the ratio of oil volume (Vo) to neat surfactant volume (Vs) and the water solubilisation ratio is the ratio of water volume (Vw) to neat surfactant volume (Vs). The intersection of Vo/Vs and Vw/Vs as salinity is varied defines a) the optimal salinity, and b) the solubilisation parameter at the optimal salinity. It has been established by Huh that IFT is inversely proportional to the square of the solubilsation parameter as described in C. Huh, "Interfacial tensions and solubilizing ability of a microemulsion phase that coexists with oil and brine, Journal of Colloid and Interface Science, September 1979, pp 408-426". When the solubilisation parameter is 10 or higher, the IFT at the optimal salinity is <0.003 dyne/cm (0.003 x 10⁻⁵ N/cm) which is required to mobilise residual oil via surfactant EOR. Thus the target solubilisation parameter for our surfactant screening is 10 or greater with the higher the value the more "active" the surfactant.

As well as indicating where ultra low IFTs are achieved the microemulsion phase test provides extra qualitative information that is relevant to a surfactant flood. This includes the relative viscosity of phases, wetting behaviour, the presence of undesirable macroemulsions or gels and the time for the phases to equilibrate (fast equilibration indicating a more promising system).

An amount of the hydrocarbon recovery composition may be added to the hydrocarbon/water mixture and an interfacial tension value for the resulting fluid may be determined. A low interfacial tension value (e.g., less than about 1 dyne/cm (1 x 10⁻⁵ N/cm)) may indicate that the composition reduced at least a portion of the surface energy between the hydrocarbons and water. Reduction of surface energy may indicate that at least a portion of the hydrocarbon/water mixture may mobilize through at least a portion of a hydrocarbon containing formation.

In an embodiment, a hydrocarbon recovery composition may be added to a hydrocarbon/water mixture and the interfacial tension value may be determined. Preferably, the interfacial tension is less than about 0.1 dyne/cm (0.1 x 10⁻⁵ N/cm). An ultralow interfacial tension value (e.g., less than about 0.01 dyne/cm (0.01 x 10⁻⁵ N/cm)) may indicate that the hydrocarbon recovery composition lowered at least a portion of the surface tension between the hydrocarbons and water such that at least a portion of the hydrocarbons may mobilize through at least a portion of the hydrocarbon containing formation. At least a portion of the hydrocarbons may mobilize more easily through at least a portion of the hydrocarbon containing formation at an ultra low interfacial tension than hydrocarbons that have been treated with a composition that results in an interfacial tension value greater than 0.01 dynes/cm (0.01 x 10⁻⁵ N/cm) for the fluids in the formation. Addition of a hydrocarbon recovery composition to fluids in a hydrocarbon containing formation that results in an ultralow interfacial tension value may increase the efficiency at which hydrocarbons may be produced. A hydrocarbon recovery composition concentration in the hydrocarbon containing formation may be minimized to minimize cost of use during production.

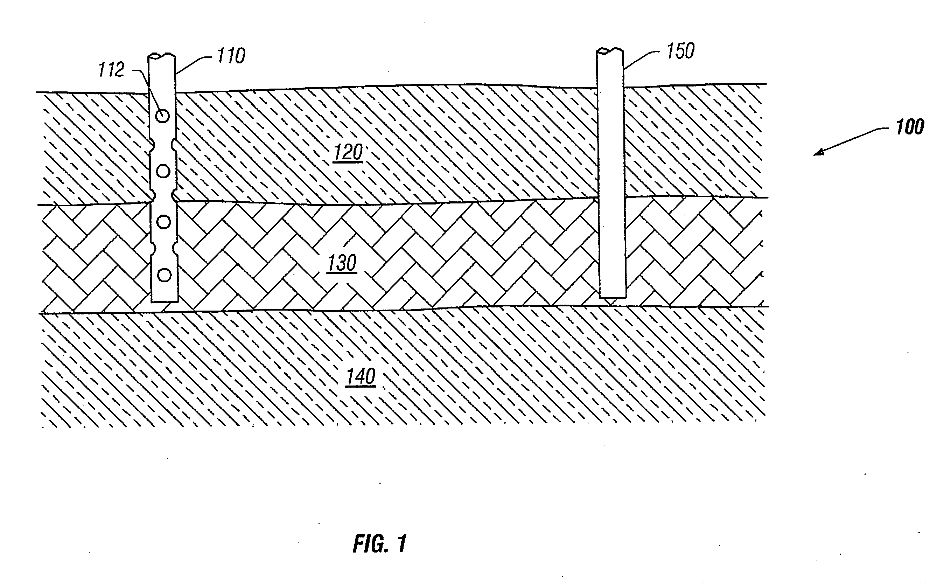

In an embodiment of a method to treat a hydrocarbon containing formation, a hydrocarbon recovery composition including a vinylidene olefin sulfonate may be provided (e.g., injected) into hydrocarbon containing formation 100 through injection well 110 as depicted in FIG. 1. Hydrocarbon formation 100 may include overburden 120, hydrocarbon layer 130, and underburden 140. Injection well 110 may include openings 112 that allow fluids to flow through hydrocarbon containing formation 100 at various depth levels. In certain embodiments, hydrocarbon layer 130 may be less than 1000 feet (304.8 m)below earth's surface. In some embodiments, underburden 140 of hydrocarbon containing formation 100 may be oil wet. Low salinity water may be present in hydrocarbon containing formation 100, in other embodiments.

A hydrocarbon recovery composition may be provided to the formation in an amount based on hydrocarbons present in a hydrocarbon containing formation. The amount of hydrocarbon recovery composition, however, may be too small to be accurately delivered to the hydrocarbon containing formation using known delivery techniques (e.g., pumps). To facilitate delivery of small amounts of the hydrocarbon recovery composition to the hydrocarbon containing formation, the hydrocarbon recovery composition may be combined with water and/or brine to produce an injectable fluid.

In an embodiment, the hydrocarbon recovery composition is provided to the formation containing crude oil with heavy components by admixing it with brine from the formation from which hydrocarbons are to be extracted or with fresh water. The mixture is then injected into the hydrocarbon containing formation.

In an embodiment, the hydrocarbon recovery composition is provided to a hydrocarbon containing formation 100 by admixing it with brine from the formation. Preferably, the hydrocarbon recovery composition comprises from about 0.01 to about 2.00 wt% of the total water and/or brine/hydrocarbon recovery composition mixture (the injectable fluid). More important is the amount of actual active matter that is present in the injectable fluid (active matter is the surfactant, here the vinylidene olefin sulfonate or the blend containing it). Thus, the amount of the vinylidene olefin sulfonate in the injectable fluid may be from about 0.05 to about 1.0 wt%, preferably from about 0.1 to about 0.8 wt%. More than 1.0 wt% could be used but this would likely increase the cost without enhancing the performance. The injectable fluid is then injected into the hydrocarbon containing formation.

The vinylidene olefin sulfonate may be used without a co-surfactant and/or a solvent. The vinylidene olefin sulfonate may not perform optimally by itself for certain crude oils. Co-surfactants and/or co-solvents may be added to the hydrocarbon recovery composition to enhance the activity.

The hydrocarbon recovery composition may interact with at least a portion of the hydrocarbons in hydrocarbon layer 130. The interaction of the hydrocarbon recovery composition with hydrocarbon layer 130 may reduce at least a portion of the interfacial tension between different hydrocarbons. The hydrocarbon recovery composition may also reduce at least a portion of the interfacial tension between one or more fluids (e.g., water, hydrocarbons) in the formation and the underburden 140, one or more fluids in the formation and the overburden 120 or combinations thereof.

In an embodiment, a hydrocarbon recovery composition may interact with at least a portion of hydrocarbons and at least a portion of one or more other fluids in the formation to reduce at least a portion of the interfacial tension between the hydrocarbons and one or more fluids. Reduction of the interfacial tension may allow at least a portion of the hydrocarbons to form an emulsion with at least a portion of one or more fluids in the formation. An interfacial tension value between the hydrocarbons and one or more fluids may be altered by the hydrocarbon recovery composition to a value of less than about 0.1 dyne/cm (0.1 x 10⁻⁵ N/cm). In some embodiments, an interfacial tension value between the hydrocarbons and other fluids in a formation may be reduced by the hydrocarbon recovery composition to be less than about 0.05 dyne/cm (0.05 x 10⁻⁵ N/cm). An interfacial tension value between hydrocarbons and other fluids in a formation may be lowered by the hydrocarbon recovery composition to less than 0.001 dyne/cm (0.001 x 10⁻⁵ N/cm), in other embodiments.

At least a portion of the hydrocarbon recovery composition/hydrocarbon/fluids mixture may be mobilized to production well 150. Products obtained from the production well 150 may include, but are not limited to, components of the hydrocarbon recovery composition (e.g., a long chain aliphatic alcohol and/or a long chain aliphatic acid salt), methane, carbon monoxide, water, hydrocarbons, ammonia, or combinations thereof. Hydrocarbon production from hydrocarbon containing formation 100 may be increased by greater than about 50% after the hydrocarbon recovery composition has been added to a hydrocarbon containing formation.

In certain embodiments, hydrocarbon containing formation 100 may be pretreated with a hydrocarbon removal fluid. A hydrocarbon removal fluid may be composed of water, steam, brine, gas, liquid polymers, foam polymers, monomers or mixtures thereof. A hydrocarbon removal fluid may be used to treat a formation before a hydrocarbon recovery composition is provided to the formation. Hydrocarbon containing formation 100 may be less than 1000 feet (304.8 m) below the earth's surface, in some embodiments. A hydrocarbon removal fluid may be heated before injection into a hydrocarbon containing formation 100, in certain embodiments. A hydrocarbon removal fluid may reduce a viscosity of at least a portion of the hydrocarbons within the formation. Reduction of the viscosity of at least a portion of the hydrocarbons in the formation may enhance mobilization of at least a portion of the hydrocarbons to production well 150. After at least a portion of the hydrocarbons in hydrocarbon containing formation 100 have been mobilized, repeated injection of the same or different hydrocarbon removal fluids may become less effective in mobilizing hydrocarbons through the hydrocarbon containing formation. Low efficiency of mobilization may be due to hydrocarbon removal fluids creating more permeable zones in hydrocarbon containing formation 100. Hydrocarbon removal fluids may pass through the permeable zones in the hydrocarbon containing formation 100 and not interact with and mobilize the remaining hydrocarbons. Consequently, displacement of heavier hydrocarbons adsorbed to underburden 140 may be reduced over time. Eventually, the formation may be considered low producing or economically undesirable to produce hydrocarbons.

In certain embodiments, injection of a hydrocarbon recovery composition after treating the hydrocarbon containing formation with a hydrocarbon removal fluid may enhance mobilization of heavier hydrocarbons absorbed to underburden 140. The hydrocarbon recovery composition may interact with the hydrocarbons to reduce an interfacial tension between the hydrocarbons and underburden 140. Reduction of the interfacial tension may be such that hydrocarbons are mobilized to and produced from production well 150. Produced hydrocarbons from production well 150 may include, in some embodiments, at least a portion of the components of the hydrocarbon recovery composition, the hydrocarbon removal fluid injected into the well for pretreatment, methane, carbon dioxide, ammonia, or combinations thereof. Adding the hydrocarbon recovery composition to at least a portion of a low producing hydrocarbon containing formation may extend the production life of the hydrocarbon containing formation. Hydrocarbon production from hydrocarbon containing formation 100 may be increased by greater than about 50% after the hydrocarbon recovery composition has been added to hydrocarbon containing formation. Increased hydrocarbon production may increase the economic viability of the hydrocarbon containing formation.

Interaction of the hydrocarbon recovery composition with at least a portion of hydrocarbons in the formation may reduce at least a portion of an interfacial tension between the hydrocarbons and underburden 140. Reduction of at least a portion of the interfacial tension may mobilize at least a portion of hydrocarbons through hydrocarbon containing formation 100. Mobilization of at least a portion of hydrocarbons, however, may not be at an economically viable rate.

In one embodiment, polymers and/or monomers may be injected into hydrocarbon formation 100 through injection well 110, after treatment of the formation with a hydrocarbon recovery composition, to increase mobilization of at least a portion of the hydrocarbons through the formation. Suitable polymers include, but are not limited to, CIBA^{®} ALCOFLOOD^{®}, manufactured by Ciba Specialty Additives (Tarrytown, New York), Tramfloc^{®} manufactured by Tramfloc Inc. (Temple, Arizona), and HE^{®} polymers manufactured by Chevron Phillips Chemical Co. (The Woodlands, Texas). Interaction between the hydrocarbons, the hydrocarbon recovery composition and the polymer may increase mobilization of at least a portion of the hydrocarbons remaining in the formation to production well 150.

The vinylidene olefin sulfonate of the composition is thermally stable and may be used over a wide range of temperatures. The hydrocarbon recovery composition may be added to a portion of a hydrocarbon containing formation 100 that has an average temperature of above about 60°C because of the high thermal stability of the vinylidene olefin sulfonate.

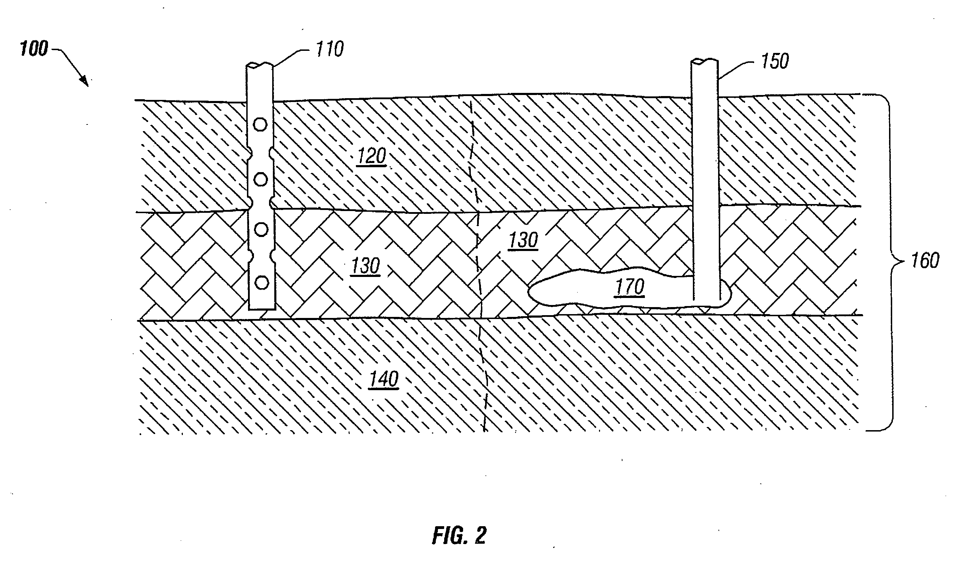

In some embodiments, a hydrocarbon recovery composition may be combined with at least a portion of a hydrocarbon removal fluid (e.g. water, polymer solutions) to produce an injectable fluid. The hydrocarbon recovery composition may be injected into hydrocarbon containing formation 100 through injection well 110 as depicted in FIG. 2. Interaction of the hydrocarbon recovery composition with hydrocarbons in the formation may reduce at least a portion of an interfacial tension between the hydrocarbons and underburden 140. Reduction of at least a portion of the interfacial tension may mobilize at least a portion of hydrocarbons to a selected section 160 in hydrocarbon containing formation 100 to form hydrocarbon pool 170. At least a portion of the hydrocarbons may be produced from hydrocarbon pool 170 in the selected section of hydrocarbon containing formation 100.

In other embodiments, mobilization of at least a portion of hydrocarbons to selected section 160 may not be at an economically viable rate. Polymers may be injected into hydrocarbon formation 100 to increase mobilization of at least a portion of the hydrocarbons through the formation. Interaction between at least a portion of the hydrocarbons, the hydrocarbon recovery composition and the polymers may increase mobilization of at least a portion of the hydrocarbons to production well 150.

In some embodiments, a hydrocarbon recovery composition may include an inorganic salt (e.g. sodium carbonate (Na₂CO₃), sodium hydroxide, sodium chloride (NaCl), or calcium chloride (CaCl₂)). The addition of the inorganic salt may help the hydrocarbon recovery composition disperse throughout a hydrocarbon/water mixture. The enhanced dispersion of the hydrocarbon recovery composition may decrease the interactions between the hydrocarbon and water interface. The use of an alkali (e.g., sodium carbonate, sodium hydroxide) may prevent adsorption of the vinylidene olefin sulfonate onto the rock surface and may create natural surfactants with components in the crude oil. The decreased interaction may lower the interfacial tension of the mixture and provide a fluid that is more mobile. The alkali may be added in an amount of from about 0.1 to 2 wt%.

Under the temperature and pressure conditions in the reservoir, a vinylidene olefin sulfonate is soluble and is effective in lowering the IFT. However, conditions above ground where the injectable fluid composition is prepared are different, i.e., lower temperature and pressure. Under such conditions and in a low salinity brine or freshwater (no salinity), the vinylidene olefin sulfonate may not be completely soluble. Before the injectable fluid can be injected, at least a significant portion of the vinylidene olefin sulfonate falls out of the mixture. Any portion of the surfactant that is not in solution, i.e. that remains insoluble and forms a waxy precipitate, will eventually plug the porous formation around the wellbore. The result will be that the injection well will plug, with the consequent loss of the ability to inject the fluid. Remedial treatments will have to be done to the well to put it back in function with the consequent loss of time and expense. It would be advantageous if a means were found to keep the vinylidene olefin sulfonate in solution in the injectable fluid as it is injected.

One method to improve the solubility of the vinylidene olefin sulfonates would be to use combinations of alpha olefins to prepare vinylidene olefin sulfonate mixtures of varying carbon tail lengths. The resulting VOS would have a range of carbon numbers and would likely provide improved aqueous solubility versus the "more pure" isomer vinylidene olefin sulfonates prepared from a single alpha olefin source. Another method is to add a minor amount of a solubilizer consisting of internal olefin sulfonate or some other highly-soluble surfactant.

The invention provides a method of injecting a hydrocarbon recovery composition comprising a vinylidene olefin sulfonate into a hydrocarbon containing formation which comprises: (a) making a solubilized vinylidene olefin sulfonate hydrocarbon recovery composition fluid by mixing a major portion of a vinylidene olefin sulfonate in fresh water or water having a brine salinity of less than about 2 wt% at a temperature of 50°C or lower and adding to the mixture a minor amount of a solubilizer which comprises a C₁₅₋₁₈ internal olefin sulfonate or a C₁₉₋₂₃ internal olefin sulfonate or mixtures thereof; and (b) injecting the solubilized vinylidene olefin sulfonate hydrocarbon recovery composition into the hydrocarbon containing formation. The weight ratio of the solubilizer to the vinylidene olefin sulfonate may be from about 10:90 to about 90:10.

In addition to improving aqueous phase solubility at low temperatures, the use of internal olefin sulfonate solubilizers will improve the ability of the vinylidene olefin sulfonate to remain in solution containing high levels of divalent ions such as calcium and magnesium as the solubility of vinylidene olefin sulfonates is noted for being much less in such solutions as compared to the solubility properties of internal olefin sulfonates.

### EXAMPLES

### Example 1

In this Example, compositions comprising vinylidene olefin sulfonates were tested to determine their performance as surfactants for chemical enhanced oil recovery purposes. Microemulsion phase tests were carried out at 90°C using aqueous solutions - containing the test surfactant at 2% active concentration and with different sodium chloride concentrations - and an alkane. The optimal salinity and solubilization ratio were determined for the vinylidene olefin sulfonates using the alkanes n-octane and n-dodecane to simulate two crude oils of different Equivalent Alkane Carbon Numbers, EACNs. Additionally, some comparative tests were performed with internal olefin sulfonates with similar carbon numbers to evaluate their relative performance. Thus a C16 VOS was compared to IOS C15-18, a C20 VOS compared to a C19-23 IOS and C20-24 IOS, and a C24 VOS compared with an IOS C24-28. The results are shown in Table 1. The interfacial tension was calculated from the solubilisation ratio using the Chun Huh equation.

**Table 1: Microemulsion phase test data**

| Sample | Alkane | Sulfonate component | Optimal Salinity (%NaCl) | Solubilization Ratio | IFT |
|---|---|---|---|---|---|
| A | Octane | C16 VOS | 3.2 | 10 | 0.003 |
| B | Octane | C15-18 IOS | 11 | 10 | 0.003 |
| C | Octane | C20 VOS | 0.15 | 35 | 0.0002 |
| D | Octane | C19-23 IOS | 3.4 | 40 | 0.0002 |
| E | Octane | C20-24 IOS | 1.6 | 40 | 0.0002 |
| F | Octane | C24 VOS | 0 | n.m. | n.m. |
| G | Octane | C24-28 IOS | 0.5 | 25 | 0.0005 |
| H | Dodecane | C16 VOS | 5.2 | 5-10 | 0.01-0.003 |
| I | Dodecane | C15-18 IOS | 17.2 | 10 | 0.003 |
| J | Dodecane | C20 VOS | 0.4 | 20 | 0.0007 |
| K | Dodecane | C19-23 IOS | 6.5 | 25 | 0.0005 |
| L | Dodecane | C24 VOS | 0.05 | n.m. | n.m. |
| M | Dodecane | C24-28 IOS | 1.6 | n.m. | n.m. |

As can be seen from the Table, the vinylidene olefin sulfonates are effective surfactants for chemical EOR, giving moderate to high solubilisation ratios (and low to ultralow IFTs) which are comparable to an internal olefin sulfonate. It is notable that the optimal salinity of a VOS is lower than a comparable IOS which means that the VOS family is more suitably used in different hydrocarbon formations with lower salinities.

The VOS family matches the salinity range of 0 to about 4% NaCl, indicating it is suitable to salinities up to about that of sea water.

During the preparation of the aqueous solutions it was noted that VOS solubility was worse than the corresponding IOS. As mentioned earlier, one method to improve the solubility of the vinylidene olefin sulfonates would be to use combinations of alpha olefins to prepare vinylidene olefin sulfonate mixures of varying carbon tail lengths. The resulting VOS would have a range of carbon numbers and would likely provide improved aqueous solubility versus the "more pure" isomer vinylidene olefin sulfonates prepared from a single alpha olefin source. Another method is to add a minor amount of a solubilizer consisting of internal olefin sulfonate or some other highly-soluble surfactant.

## Claims

1. A method of treating a formation containing crude oil, comprising:
(a) providing a hydrocarbon recovery composition to at least a portion of the crude oil containing formation, wherein the composition comprises at least 10 wt% of vinylidene olefin sulfonate, and wherein the hydrocarbon recovery composition is provided to the crude oil containing formation by first admixing it with water and/or brine from the formation from which crude oil is to be extracted to form an injectable fluid, wherein the vinylidene olefin sulfonate comprises from 0.05 to 1.0 wt%, preferably from 0.1 to 0.8 wt% of the injectable fluid, and then injecting the injectable fluid into the formation; and
(b) allowing the composition to interact with hydrocarbons in the crude oil containing formation; and
wherein the formation is treated with a polymer and/or monomer.

## Patentansprüche

1. Verfahren zur Behandlung einer Rohöl enthaltenden Formation, umfassend:
(a) Bereitstellen einer Kohlenwasserstoff-Rückgewinnungszusammensetzung für wenigstens einen Teil der Rohöl enthaltenden Formation, wobei die Zusammensetzung wenigstens 10 Gew.-% Vinylidenolefinsulfonat umfasst, und wobei die Kohlenwasserstoff-Rückgewinnungszusammensetzung der Rohöl enthaltenden Formation bereitgestellt wird, indem sie zuerst mit Wasser und/oder Sole aus der Formation, von der Rohöl extrahiert werden soll, vermischt wird, um ein einspritzbares Fluid zu bilden, wobei das Vinylidenolefinsulfonat von 0,05 bis 1,0 Gew.-% beträgt, vorzugsweise von 0,1 bis 0,8 Gew.-% des einspritzbaren Fluids, und dann das einspritzbare Fluid in die Formation eingespritzt wird; und
(b) Zulassen, dass die Zusammensetzung mit Kohlenwasserstoffen in der Rohöl enthaltenden Formation zusammenwirkt; und
wobei die Formation mit einem Polymer und/oder Monomer behandelt wird.

## Revendications

1. Procédé de traitement d'une formation contenant du pétrole brut, comprenant :
(a) la fourniture d'une composition de récupération d'hydrocarbure à au moins une partie de la formation contenant du pétrole brut, dans lequel la composition comprend au moins 10% en poids d'oléfine-sulfonate de vinylidène, et dans lequel la composition de récupération d'hydrocarbure est fournie à la formation contenant du pétrole brut en la mélangeant tout d'abord avec de l'eau et/ou une solution saline de la formation à partir de laquelle le pétrole brut doit être extrait pour former un fluide injectable, dans lequel l'oléfine-sulfonate de vinylidène comprend de 0,05 à 1,0% en poids, de préférence de 0,1 à 0,8% en poids de fluide injectable, puis en injectant le fluide injectable dans la formation ; et
(b) l'interaction de la composition avec des hydrocarbures dans la formation contenant du pétrole brut ; et
dans lequel la formation est traitée avec un polymère et/ou un monomère.
